# EUROPEAN PATENT APPLICATION

(11) **EP 2 730 218 A1**
(43) Date of publication of application: **14.05.2014**
(21) Application number: 13191986.2
(22) Date of filing: 07.11.2013
(51) Int. Cl.: A61B 5/00, A61B 5/0205, A61B 5/024, A43B 3/00

(54) **Footwear able to be wirelessly charged and transmit bio information, method for managing health in wireless communication system including the footwear, and wireless communication system to which the method is applied**

(30) Priority: 09.11.2012 KR 20120126930
(71) Applicant: Hanrim Postech Co., Ltd, Suwon-si, Gyeonggi-do (KR)
(72) Inventor: Jung, Chun-Kil, Seoul (KR)
(74) Representative: Zardi, Marco

(57) **Abstract**

Disclosed herein are a method for managing health in a wireless communication system including a footwear to which a wireless charging technology is applied, including obtaining a bio information of a user from a bio information detecting sensor of the footwear using a power obtained through a wireless power receiving device installed therein; and transmitting the bio information to an external terminal via a communication module, a footwear able to be wirelessly charged and transmit a bio information, and a wireless communication system to which the method is applied.

## Description

### BACKGROUND

### Field

The present invention relates to a footwear able to be wirelessly charged and transmit a bio information, a method for managing health in a wireless communication system including the footwear, and a wireless communication system to which the method is applied.

### Description of the Related Art

In accordance with the development of a medical technology, an effort to monitor a bio signal of a patient at a remote place has been continuously made. For example, an electrocardiogram monitoring system for diagnosing a disease and a state associated with a heart guides an electrocardiogram waveform for a short period of time. Therefore, in order to observe a heart problem such as an arrhythmia that is intermittently generated, it has been required to continuously record and analyze th e heart problem for a long period of time.

In addition, any means capable of coping with a rapid risk generated due to sleep apnea, or the like, in a home, or the like have not been suggested.

According to the related art, it is impossible to measure a bio signal of a patient in real time and an action may not be immediately taken when the patient is in an emergency state. In order to solve these problems, various studies and developments have been continuously conducted.

Korean Patent Application No. 10-1995-0029913 (entitled "System of Monitoring Bio Signal in Real Time by Using Wireless Communication Network; hereinafter, referred to as the 'related patent technology') has disclosed a technology capable of diagnosing and preventing a disease of a patient by continuously checking a bio signal of the patient via a bio signal measuring device to transmit a signal indicating that there is an abnormality in the bio signal to a bio signal monitoring server device of a hospital in real time via a wireless communication network only when it is indicated that there is the abnormality in the bio signal and transmitting a diagnosing and emergency treating method from a bio signal monitoring center of the hospital to the bio signal measuring device of the patient in real time.

### SUMMARY

An object of the present invention is to provide a method for managing health in a wireless communication system including a footwear to which a wireless charging technology is applied, capable of obtaining a bio information by the footwear worn by a user for a long period time and managing health and coping with an emergency situation through the bio information, and a wireless communication system to which the method is applied.

According to an exemplary embodiment of the present invention, there is provided a method for managing health in a wireless communication system including a footwear to which a wireless charging technology is applied, the method including: obtaining a bio information of a user from a bio information detecting sen sor of the footwear using a power obtained through a wireless power receiving device installed therein; and transmitting the bio information to an external terminal via a communication module.

The method may further include storing an ID information in a memory installed in the footwear, wherein the transmitting of the bio information to the external terminal via the communication module includes: transmitting the ID information to the external terminal via the communication module; and transmitting the bio information to the external terminal via the communication module when an ID confirmation signal is received from the external terminal.

The external terminal may be a mobile communication terminal having a terminal ID information stored in a storing unit thereof, and the method may further include transmitting the terminal ID information and the bio information to a health management server via a mobile communication module of the mobile communication terminal.

The bio information may include a pulse information, a blood pressure information, a body temperature information, an exercising amount information, and a time information.

The method may further include: receiving an alarm information generated through the bio information via the communication module of the footwear; and outputting an alarm sound to a sound output module installed in the footwear, based on the alarm information.

According to another exemplary embodiment of the present invention, there is provided a method for managing health in a wireless communication system including a footwear to which a wireless charging technology is applied, the method including: receiving a bio information and an ID information of a user from a bio information detecting sensor of the footwear using a power obtained via a wireless power receiving device installed therein; analyzing the bio information to generate an alarm information; and transmitting the alarm information to an external terminal.

The analyzing of the bio information to generate the alarm information may include: storing a personal data base in a memory; obtaining a health record information of the user in the personal data base by using the ID information of the user; and generating the alarm information by using the health record information and the bio information.

According to still another exemplary embodiment of the present invention, there is provided a footwear able to be wirelessly charged and transmit a bio information, the footwear including: an insole; an outer cover installed on the insole and having a wear space formed therein; a heel attached to a lower surface of the insole; a secondary coil installed in any one of the insole, the outer cover, and the heel and receiving a wireless power signal from an external device; a battery installed in any one of the insole, the outer cover, and the heel and charged with an induced electromotive force generated in the secondary coil; a bio information detecting sensor installed in the wear space; a communication module installed at any one of the insole, the outer cover, and the heel; and a controller configured to charge the battery by using the wireless power signal received via the secondary coil and transmit the bio information obtained from the bio information detecting sensor to an external electronic device via the communication module.

The footwear may further include a memory configured to store an ID information of a user, wherein the controller is configured to transmit the ID information to an external terminal and transmit the bio information to the external terminal via the communication module when an ID confirmation signal is received from the external terminal via the communication module.

The bio information may include a pulse information, a blood pressure information, a body temperature information, an exercising amount information, and a time information.

The controller may be configured to output an alarm sound to a sound output module installed in the footwear based on an alarm information generated through the bio information when the alarm information is received via the communication module of the footwear.

The footwear may further include a position information module, wherein the controller is configured to transmit a position information obtained from the position information module to the external electronic device via the communication module when the alarm information is received via the communication module.

### BRIEF DESCRIPTION OF THE DRAWINGS

FIG. 1 is a view for describing a wireless communication system associated with a method for managing health in a wireless communication system according to an exemplary embodiment of the present invention;
FIG. 2 is a perspective view showing an example of a footwear able to be wirelessly charged and transmit a bio information according to the exemplary embodiment of the present invention;
FIG. 3 is a cross-sectional view of the footwear able to be wirelessly charged and transmit a bio information shown in FIG. 2;
FIG. 4 is a block diagram for describing electronic components of the footwear able to be wirelessly charged and transmit a bio information according to the exemplary embodiment of the present invention; and
FIG. 5 is a flow chart for describing the method for managing health in a wireless communication system according to the exemplary embodiment of the present invention.

### DETAILED DESCRIPTION

Hereinafter, a footwear able to be wirelessly charged and transmit a bio information, a method for managing health in a wireless communication system including the footwear, and a wireless communication system to which the method is applied according to an exemplary embodiment of the present invention will be described in detail with reference to the accompanying drawings.

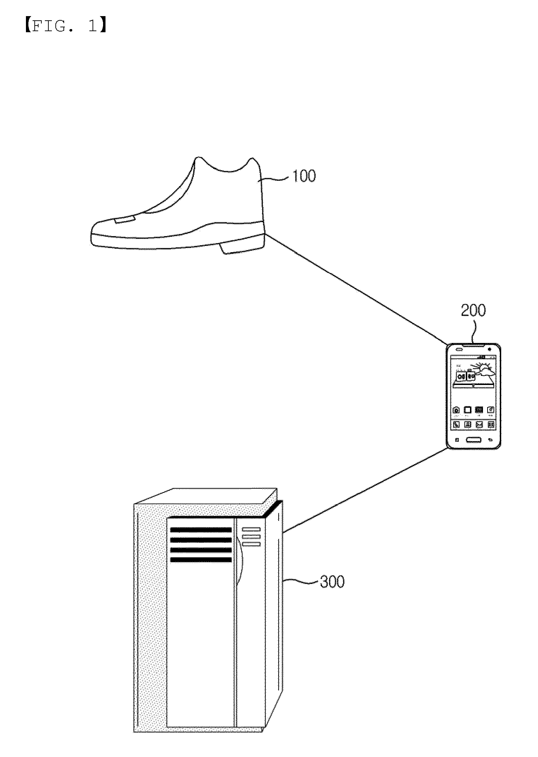

FIG. 1 is a view for describing a wireless communication system associated with a method for managing health according to an exemplary embodiment of the present invention. As shown in FIG. 1, the wireless communication system associated with the method for managing health according to the exemplary embodiment of the present invention is configured to include a footwear 100, a mobile communication terminal 200, and a health management server 300.

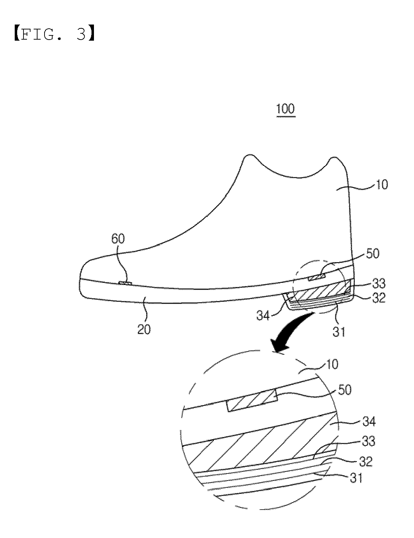

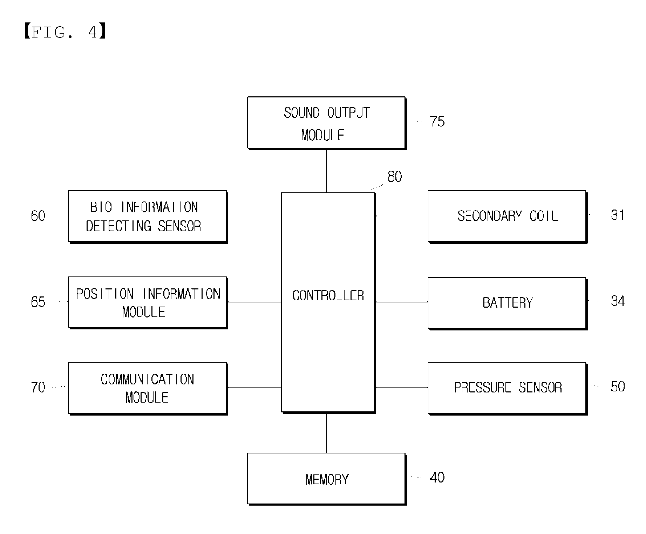

The footwear 100 transmits a bio information obtained through a bio information detecting sensor 60 (See FIGS. 3 and 4) installed therein to the mobile communication terminal 200, and the mobile communication terminal 200 obtaining the bio information transmits the bio information to the health management server 300 via a mobile communication module installed therein. The mobile communication terminal 200 and the footwear 100 communicate with each other via Bluetooth or Zigbee, which is a near field communication module.

Meanwhile, the health management server 300 obtaining the bio information confirms a health state of a user by using the bio information and periodically transmits a current health state to the mobile communication terminal. Meanwhile, in the case of an emergency situation, that is, in the case in which a blood pressure rapidly becomes high or low or a body temperature rapidly becomes higher than a set temperature, the health management server 300 transmits an alarm information to the mobile communication terminal 200.

Although an example in which the bio information is transmitted to the health management server 300 via the mobile communication terminal 200 has been described in the exemplary embodiment of the present invention, the present invention is not limited thereto. That is, the bio information may be directly transmitted from the footwear 100 to the health management server 300. That is, in the case in which a mobile communication module rather than the near field communication module is included in the footwear, the bio information may be directly transmitted together with an ID information stored in a memory of the footwear 100 to the health management server 300.

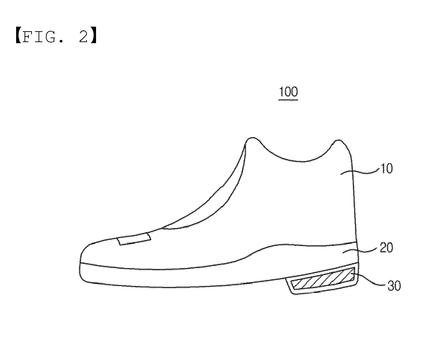

Hereinafter, a footwear 100 able to be wirelessly charged and transmit a bio information according to the exemplary embodiment of the present invention will be described with reference to FIGS. 2 to 4.

FIG. 2 is a perspective view showing an example of a footwear able to be wirelessly charged and transmit a bio information according to the exemplary embodiment of the present invention; and FIG. 3 is a cross-sectional view of the footwear able to be wirelessly charged and transmit a bio information shown in FIG. 2. As shown in FIGS. 2 and 3, the footwear 100 able to be wirelessly charged and transmit a bio information according to the exemplary embodiment of the present invention is configured to include an insole 20; an outer cover 10 formed on the insole 20; and a heel 30 attached to a rear part of the insole 20. As shown in FIG. 3, a bio information detecting sensor 60 for obtaining a bio information of a user is attached to the insole 20, and a wireless power receiving device and a battery 34 are installed in the heel. That is, the heel 30 is provided with a secondary coil 31 receiving a wireless power signal, the battery 34 charged with power from the secondary coil 31, a controller 80 (See FIG. 4) controlling the battery 34 to control an operation of the bio information detecting sensor 60, a board 33 on which a communication module 70 (See FIG. 4) for transmitting a bio information obtained by the bio information detecting sensor 60 to a mobile communication terminal, which is an external electronic device, is installed, and a shielding plate 32 installed on the secondary coil 31 and allowing a leaked magnetic field not to affect the board 33 or the battery 34.

Here, the secondary coil 31 may include a first sub coil and a second sub coil installed on the first sub coil so as to be overlapped with the first sub coil, in order to increase a transmission efficiency. That is, in the case in which a cross-sectional width of the heel is not wide, such that it is difficult to form the secondary coil 31 having a sufficient size, two coils are installed to be overlapped with each other, thereby making it possible to increase a reception efficiency of the wireless power signal.

In addition, a pressure sensor 50 may be attached to a portion corresponding to the heel to control a turn on/off of the bio information detecting sensor 60. That is, through the pressure sensor 50, an operation of the bio information detecting sensor 60 may start when it is judged that the user is in the state in which he/she wears the footwear 100 and stop when it is judged that the user is in the state in which he/she takes off the footwear 100 (that is, when a pressure is not detected). Alternatively, the controller 80 may operate the bio information detecting sensor 60 at a predetermined period based on a signal from the pressure sensor 50.

Meanwhile, a flexible circuit board may be used in order to connect the board 33 and the bio information detecting sensor 60 to each other. When a person walks or runs, the insole 20 of the footwear 100 is bent. In this case, when the flexible circuit board is used as an electrical connection means between the bio information detecting sensor 60 and the board 33, even though the insole 20 is bent, durability is not affected at all.

FIG. 4 is a block diagram for describing electronic components of the footwear able to be wirelessly charged and transmit a bio information according to the exemplary embodiment of the present invention. As shown in FIG. 4, the footwear 100 able to be wirelessly charged and transmit a bio information according to the exemplary embodiment of the present invention may be configured to include the secondary coil 31, the battery 34, a memory 40, the pressure sensor 50, the bio information detecting sensor 60, a position information module 65, a communication module 70, a sound output module 75, and the controller 80. A description of the above-mentioned contents in operations of these components will be omitted for simplification.

As shown in FIG. 4, according to the exemplary embodiment of the present invention, an ID information of a user is stored in the memory 40. The ID information of the user may be generated at the time of purchasing the footwear or be generated by using the mobile communication terminal 200 of the user. The ID information of the user is transmitted together with the bio information to the mobile communication terminal 200 via the communication module 70. In other words, when the ID information of the user is transmitted, the mobile communication terminal 200 transmits an ID confirmation signal to the footwear 100 based on whether or not an ID information of the terminal and the ID information of the user are matched to each other. The footwear 100 receiving the ID confirmation signal transmits the bio information to the mobile communication terminal 200.

Meanwhile, the footwear 100 further includes the sound output module 75 and the position information module 65. After the bio information is transmitted to the health management server 300 in the above-mentioned scheme and the health management server 300 performs an analysis by using the bio information, when it is judged that the user is in an emergency situation, an alarm information is generated and is received by the footwear 100. In this case, the controller 80 may operate the sound output module 75 to output an alarm sound, thereby asking peoples around the user for help. In addition, the controller 80 may transmit an emergency rescue request signal together with a position information obtained through the position information module 65 to an emergency rescue server via the mobile communication terminal 200. The emergency rescue server receiving the emergency rescue request sig nal may dispatch an emergency rescue team by using the position information.

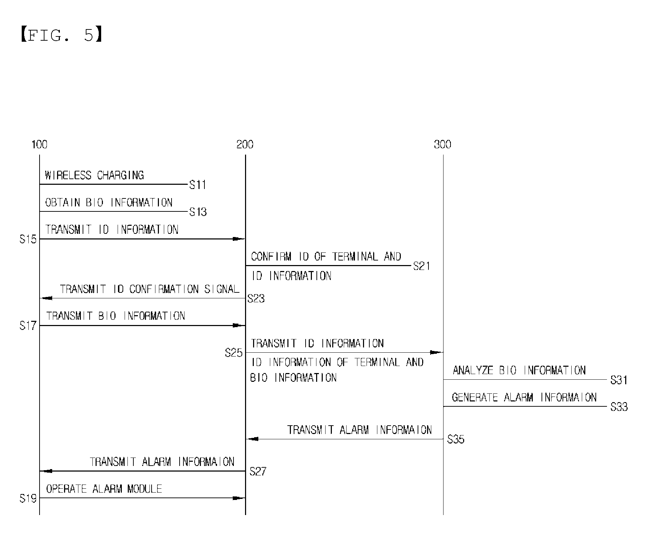

Hereinafter, a method for managing health in a wireless communication system including the footwear 100 able to be wirelessly charged and transmit a bio information and having the above-mentioned configuration will be described in detail with reference to FIG. 5.

FIG. 5 is a flow chart for describing the method for managing health in a wireless communication system according to the exemplary embodiment of the present invention. As shown in FIG. 5, first, the bio information of the user is obtained from the bio information detecting sensor 60 of the footwear 100 by using wireless power obtained through the wireless power receiving device installed therein (S11 and S13). Meanwhile, the ID information of the user is stored in the memory 40 installed in the footwear 100. The ID information of the user stored in the memory 40 is transmitted to the mobile communication terminal 200 via the communication module 70 (S15). The mobile communication terminal 200 confirms whether the ID information of the user and the ID information of the terminal are matched to each other and transmits an ID confirmation signal to the footwear 100 when they are matched to each other (S21 and S23). Then, the bio information is transmitted to the external terminal 200 (mobile communication terminal) via the communication module 70 (S17). The mobile communication terminal 200 obtaining the bio information transmits the ID information of the terminal (or the ID information of the user) and the bio information to the health management server 300 via the mobile communication module installed therein (S25).

Here, the bio information may include a pulse information, a blood pressure information, a body temperature information, an exercising amount information, and a time information. The health management server 300 analyzing the bio information periodically transmits a health state information to the mobile communication terminal 200.

Here, a personal database is stored in a memory of the health management server 300, and the health management server 300 obtains a health record information of the user from the personal database by using the ID information and performs a health analysis by using the health record information and the bio information. When it is judged by the health analysis based on the bio information that the user is in an emergency situation, the health management server 300 generates the alarm information (S31 and S33). The generated alarm information is transmitted to the footwear 100 via the mobile communication terminal (S35 and S27).

The footwear 100 receiving the alarm information operates an alarm module 75 (sound output module) installed therein to output an audio for asking persons around the user for help and operates the position information module 65 to obtain the position information and transmit the obtained position information to the emergency rescue server, thereby requesting the emergency rescue team.

According to the exemplary embodiment of the present invention having the above-mentioned configuration, since the battery is charged by a wireless charging technology, a completely waterproof footwear may be manufactured at a cheap cost and a difficulty due to replacement of the battery may be prevented.

In addition, according to the exemplary embodiment of the present invention, since the bio information for a long period of time may be obtained from the footwear worn for a long period of time, basic data through which a health state of the user may be more accurately judged may be obtained.

Further, according to the exemplary embodiment of the present invention, when it is judged that the health state of the user is an emergency state, the user may be rescued by using the position information of the footwear.

In the footwear able to be wirelessly charged and transmit a bio information, the method for managing health in a wireless communication system including the footwear, and the wireless communication system to which the method is applied according to the exemplary embodiment of the present invention as described above, the configurations and the methods of the above-mentioned exemplary embodiments are not restrictively applied. That is, all or some of the respective exemplary embodiments may be selectively combined with each other so that they may be variously modified.

## Claims

1. A method for managing health in a wireless communication system including a footwear to which a wireless charging technology is applied, th e method comprising:
obtaining a bio information of a user from a bio information detecting sensor of the footwear using a power obtained through a wireless power receiving device installed therein; and
transmitting the bio information to an external terminal via a communication module.

2. The method of claim 1, further comprising:
storing an ID information in a memory installed in the footwear,
wherein the transmitting of the bio information to the external terminal via the communication module includes:
transmitting the ID information to the external terminal via the communication module; and
transmitting the bio information to the external terminal via the communication module when an ID confirmation signal is received from the external terminal.

3. The method of claim 2, wherein the external terminal is a mobile communication terminal having a terminal ID information stored in a storing unit thereof, and
the method further comprises transmitting the terminal ID information and the bio information to a health management server via a mobile communication module of the mobile communication terminal.

4. The method of claim 1, wherein the bio information includes a pulse information, a blood pressure information, a body temperature information, an exercising amount information, and a time information.

5. The method of claim 1, further comprising:
receiving an alarm information generated through the bio information via the communication module of the footwear; and
outputting an alarm sound to a sound output module installed in the footwear, based on the alarm information.

6. A method for managing health in a wireless communication system including a footwear to which a wireless charging technology is applied, the method comprising:
receiving a bio information and an ID information of a user from a bio information detecting sensor of the footwear using a power obtained via a wireless power receiving device installed therein;
analyzing the bio information to generate an alarm information; and
transmitting the alarm information to an external terminal.

7. The method of claim 6, wherein the analyzing of the bio information to generate the alarm information includes:
storing a personal database in a memory;
obtaining a health record information of the user in the personal database by using the ID information of the user; and
generating the alarm information by using the health record information and the bio information.

8. A footwear able to be wirelessly charged and transmit a bio information, the footwear comprising:
an insole;
an outer cover installed on the insole and having a wear space formed therein;
a heel attached to a lower surface of the insole;
a secondary coil installed in any one of the insole, the outer cover, and the heel and receiving a wireless power signal from an external device;
a battery installed in any one of the insole, the outer cover, and the heel and charged with an induced electromotive force generated in the secondary coil;
a bio information detecting sensor installed in the wear space;
a communication module installed at any one of the insole, the outer cover, and the heel; and
a controller configured to charge the battery by using the wireless power signal received via the secondary coil and transmit the bio information obtained from the bio information detecting sensor to an external electronic device via the communication module.

9. The footwear of claim 8, further comprising a memory configured to store an ID information of a user,
wherein the controller is configured to transmit the ID information to an external terminal and transmit the bio information to the external terminal via the communication module when an ID confirmation signal is received from the external terminal via the communication module.

10. The footwear of claim 8, wherein the bio information includes a pulse information, a blood pressure information, a body temperature information, a exercising amount information, and a time information.

11. The footwear of claim 8, wherein the controller is configured to output an alarm sound to a sound output module installed in the footwear based on an alarm information generated through the bio information when the alarm information is received via the communication module of the footwear.

12. The footwear of claim 11, further comprising a position information module,
wherein the controller is configured to transmit a position information obtained from the position information module to the external electronic device via the communication module when the alarm information is received via the communication module.
